# EUROPÄISCHE PATENTANMELDUNG

(11) **EP 2 383 256 A1**
(43) Veröffentlichungstag der Anmeldung: **02.11.2011**
(21) Anmeldenummer: 10160742.2
(22) Anmeldetag: 22.04.2010
(51) Int. Cl.: C07C 315/00, C07C 317/14

(54) **Verfahren zur Herstellung von 4,4'-Dichlordiphenylsulfon**

(71) Anmelder: BASF SE, 67056 Ludwigshafen (DE)
(72) Erfinder: Die Erfindernennung liegt noch nicht vor

(57) **Zusammenfassung**

Die Erfindung betrifft ein Verfahren zur Herstellung von 4,4'-Dichlordiphenylsulfon ausgehend von Monochlorbenzol, wobei der Gehalt des eingesetzten Monochlorbenzols an Kohlenwasserstoffen mit von 5 bis 8 Kohlenstoffatomen höchstens 100 ppm, bezogen auf das Gesamtgewicht des eingesetzten Monochlorbenzols einschließlich der Nebenkomponenten, beträgt.

Darüber hinaus betrifft die vorliegende Erfindung die Verwendung von Monochlorbenzol mit den genannten Eigenschaften zur Herstellung von 4,4'-Dichlordiphenylsulfon.

## Beschreibung

Die Erfindung betrifft ein Verfahren zur Herstellung von 4,4'-Dichlordiphenylsulfon ausgehend von Monochlorbenzol, wobei der Gehalt des eingesetzten Monochlorbenzols an Kohlenwasserstoffen mit von 5 bis 8 Kohlenstoffatomen höchstens 100 ppm, bezogen auf das Gesamtgewicht des eingesetzten Monochlorbenzols einschließlich der Nebenkomponenten, beträgt.

Darüber hinaus betrifft die vorliegende Erfindung die Verwendung von Monochlorbenzol mit den genannten Eigenschaften zur Herstellung von 4,4'-Dichlordiphenylsulfon.

4,4'-Dichlordiphenylsulfon wird insbesondere als Monomer bei der Synthese von Polyarylenethersulfonen eingesetzt. Kommerziell bedeutende Beispiele sind Polyethersulfon (Polymerisation von 4,4'-Dihydroxydiphenysulfon mit 4,4'-Dichlordiphenylsulfon), Polysulfon (Polymerisation von Bisphenol A mit 4,4'-Dichlordiphenylsulfon) und Polyphenylensulfon (Polymerisation von 4,4'-Dihydroxybiphenyl mit 4,4'-Dichlordiphenylsulfon). 4,4'-Dichlordiphenylsulfon ist damit ein zentraler Baustein für die Herstellung dieser technischen Kunststoffe.

Als Edukt für die Herstellung von Polyarylenethersulfonen wird hochreines 4,4'-Dichlordiphenylsulfon bevorzugt, einerseits da ausschließlich aus dem 4,4'-Isomer lineare, nicht gewinkelte Polymere entstehen, die die gewünschten Produkteigenschaften, wie z.B. Chemikalien- und Temperaturbeständigkeit, hohe Dimensionsstabilität und Schwerentflammbarkeit aufweisen, andererseits, da Verunreinigungen häufig zu unerwünschten Verfärbungen und zur Verschlechterung der Eigenschaften der Polymere führen.

Verfahren zur Herstellung von 4,4'-Dichlordiphenylsulfon ausgehend von Monochlorbenzol sind aus dem Stand der Technik bekannt. Die bekannten Verfahren umfassen insbesondere die Herstellung ausgehend von Monochlorbenzol und einem Sulfonierungsmittel über 4-Chlorbenzolsulfonsäure als Zwischenprodukt, welches im Allgemeinen nicht isoliert wird.

Die US 2,593,001 beschreibt ein kontinuierliches Verfahren zur Herstellung von Diarylsulfonen durch Reaktion von aromatischen Sulfonsäuren mit Aromaten, wobei das Reaktionswasser durch die im Gegenstrom gasförmig zugegebene aromatische Verbindung kontinuierlich aus der Reaktionszone entfernt wird.

Die US 2,971,985 offenbart die Synthese von Dichlordiphenylsulfon unter Verwendung von SO₃, Dimethylsulfat und Monochlorbenzol.

Reaktionsausträge aus der Synthese von 4,4'-Dichlordiphenylsulfon nach US 2,593,001 oder US 2,971,985 sind üblicherweise gefärbt. Diese Färbung durch stark farbgebende Nebenprodukte in sehr geringen Konzentrationen kann mittels Aufarbeitung durch Kristallisation nur unvollständig oder nur in sehr aufwendiger Weise verhindert werden.

Um 4,4'-Dichlordiphenylsulfon in einer für die Anwendung als Polymerbaustein notwendigen Qualität zu erhalten, ist grundsätzlich eine Aufarbeitung des zunächst erhaltenen Rohproduktes, d. h. einer Mischung enthaltend 4,4'-Dichlordiphenylsulfon, erforderlich. Hierzu sind aus dem Stand der Technik unterschiedliche Verfahren bekannt.

Die US 4,937,387 beschreibt, aufbauend auf der Synthese gemäß US 2,593,001, die Auftrennung des Reaktionsgemischs durch Zugabe von Wasser, Trennung der beiden entstehenden flüssigen Phasen und anschließend Isolierung von Dichlordiphenylsulfon.

Gemische der Dichlordiphenylsulfon-Isomere können z.B. durch Kristallisation mit beziehungsweise aus Alkoholen aufgearbeitet werden, so dass man erhöhte Reinheiten des gewünschten 4,4'-Dichlordiphenylsulfon erhält. In der EP-A 279387 wird diese Art der Reinigung durch Umkristallisation beschrieben.

Nachteilig an den bekannten Verfahren zur Herstellung von 4,4'-Dichlordiphenylsulfon ist somit die Komplexität der Aufarbeitung, die sich an die Gewinnung des zunächst erhaltenen Rohproduktes anschließt. Der Grad der Komplexität wird in hohem Maße durch die Art und Menge an Nebenkomponenten bestimmt. Nebenkomponenten von 4,4'-Dichlordiphenylsulfon lassen sich üblicherweise durch Kristallisation nur schwer abtrennen. Erschwerend kommt hinzu, dass farbgebende Nebenkomponenten bereits in sehr geringen Mengen das 4,4'-Dichlordiphenylsulfon und gegebenenfalls daraus hergestellte Polymere in unerwünschter Weise verfärben. Es wäre somit wünschenswert, ein Verfahren zur Herstellung von 4,4'-Dichlordiphenylsulfon bereitzustellen, welches die Bildung von farbgebenden Nebenkomponenten von 4,4'-Dichlordiphenylsulfon vermindert oder vermeidet.

Es war somit die Aufgabe der vorliegenden Erfindung, ein Verfahren zur Herstellung von 4,4'-Dichlordiphenylsulfon zur Verfügung zu stellen, das die vorgenannten Nachteile nicht oder in geringerem Umfang aufweist.

Insbesondere sollte das Verfahren in verfahrenstechnisch einfacher Weise 4,4'-Dichlordiphenylsulfon in hoher Reinheit zur Verfügung zu stellen. Der Anteil an bei der Herstellung gebildeten Nebenkomponenten von 4,4'-Dichlordiphenylsulfon, insbesondere farbgebenden Nebenkomponenten, sollte gegenüber dem Stand der Technik reduziert oder sogar vermieden werden.

Die vorgenannten Aufgaben werden gelöst durch das erfindungsgemäße Verfahren zur Herstellung 4,4'-Dichlordiphenylsulfon. Bevorzugte Ausführungsformen sind den Ansprüchen und der nachfolgenden Beschreibung zu entnehmen. Kombinationen bevorzugter Ausführungsformen verlassen den Rahmen der vorliegenden Erfindung nicht.

Das erfindungsgemäße Verfahren zur Herstellung von 4,4'-Dichlordiphenylsulfon erfolgt ausgehend von Monochlorbenzol, wobei der Gehalt des eingesetzten Monochlorbenzols an Kohlenwasserstoffen mit von 5 bis 8 Kohlenstoffatomen höchstens 100 ppm, jeweils bezogen auf das Gesamtgewicht des eingesetzten Monochlorbenzols einschließlich der Nebenkomponenten, beträgt.

Der Gehalt des eingesetzten Monochlorbenzols an Kohlenwasserstoffen mit von 5 bis 8 Kohlenstoffatomen wird im Rahmen der vorliegenden Erfindung grundsätzlich als Gewichtsmenge der Kohlenwasserstoffe (in der Einheit ppm, die im Rahmen der vorliegenden Erfindung Gewichtsteile kennzeichnet) berechnet und auf das Gesamtgewicht des eingesetzten Monochlorbenzols einschließlich aller Nebenkomponenten bezogen.

Der Gehalt des eingesetzten Monochlorbenzols an Kohlenwasserstoffen wird im Rahmen der vorliegenden Erfindung mittels gaschromatographischer Trennung und anschließender Detektion mittels Flammenionisation bestimmt. Die quantitative Bestimmung der Nebenkomponenten erfolgt dabei über die Addition eines Standards. Entsprechende quantitative Verfahren sind dem Fachmann bekannt. Die üblicherweise erreichbare Nachweisgrenze dieser Methode liegt bei 0,1 ppm.

Grundsätzlich kommen im Rahmen des erfindungsgemäßen Verfahrens alle bekannten Verfahren zur Herstellung von 4,4'-Dichlordiphenylsulfon in Betracht, die von Monochlorbenzol ausgehen, insbesondere Verfahren, welche die Umsetzung von Monochlorbenzol mit einem Sulfonierungsmittel umfassen, insbesondere solche, die über 4-Chlorbenzolsulfonsäure als Zwischenprodukt verlaufen. Entsprechende Verfahren sind dem Fachmann bekannt. Bevorzugt verwendete Verfahren zur Herstellung von 4,4'-Dichlordiphenylsulfon werden im Rahmen von Schritt (b) einer bevorzugten Ausführungsform weiter unten beschrieben.

Vorzugsweise weist das eingesetzte Monochlorbenzol gleichzeitig eine Reinheit von mindestens 99,8 Gew.-% auf. Eine Reinheit von mindestens 99,8 Gew.-% bedeutet im Rahmen der vorliegenden Erfindung, dass das eingesetzte Monochlorbenzol mindestens zu 99,8 Gew.-% aus der chemischen Verbindung Monochlorbenzol besteht. Die Angabe der Gew.-% bezieht sich auf das Gesamtgewicht des eingesetzten Monochlorbenzols. Es ist dem Fachmann bekannt, dass das eingesetzte Monochlorbenzol neben der chemischen Verbindung Monochlorbenzol noch sogenannte Verunreinigungen, im Folgenden Nebenkomponenten genannt, enthält. Die Angabe der Reinheit bezieht sich somit grundsätzlich auf das Gesamtgewicht des eingesetzten Monochlorbenzols und umfasst sämtliche Nebenkomponenten einschließlich der genannten Kohlenwasserstoffe.

Gemäß der vorliegenden Erfindung beträgt der Anteil an Nebenkomponenten im bei der Herstellung von 4,4'-Dichlordiphenylsulfon eingesetzten Monochlorbenzol vorzugsweise höchstens 0,2 Gew.-%, bezogen auf das Gesamtgewicht des eingesetzten Monochlorbenzols einschließlich aller Nebenkomponenten. Der Anteil von Kohlenwasserstoffen, die definitionsgemäß ausschließlich aus Kohlenstoff und Wasserstoff bestehen, mit 5 bis 8 Kohlenstoffatomen am Gesamtgewicht des eingesetzten Monochlorbenzols beträgt erfindungsgemäß höchstens 100 ppm.

Vorzugsweise beträgt der Anteil von Kohlenwasserstoffen mit 5 bis 8 Kohlenstoffatomen am Gesamtgewicht des eingesetzten Monochlorbenzols insgesamt höchstens 80 ppm, besonders bevorzugt höchstens 50 ppm, insbesondere höchstens 20 ppm, ganz besonders bevorzugt höchstens 10 ppm, insbesondere höchstens 5 ppm, jeweils bezogen auf das Gesamtgewicht des eingesetzten Monochlorbenzols einschließlich der Nebenkomponenten.

Im Rahmen der vorliegenden Erfindung ist die untere Grenze für die erfindungsgemäßen Kohlenwasserstoffe mit 5 bis 8 Kohlenstoffatomen an sich Null. Allerdings ist es nicht oder nur mit sehr hohem Aufwand möglich, die untere Grenze von Null zu erreichen. Insofern ist eine übliche, verfahrenstechnisch bedingte untere Grenze für den Anteil von Kohlenwasserstoffen mit 5 bis 8 Kohlenstoffatomen am Gesamtgewicht des eingesetzten Monochlorbenzols beispielsweise 0,1 ppm beziehungsweise 0,01 ppm, bezogen auf das Gesamtgewicht des eingesetzten Monochlorbenzols einschließlich der Nebenkomponenten. Zur Bestimmung von Gehalten an Nebenkomponenten, die mit weniger als 0,1 ppm enthalten sind, wendet der Fachmann bekannte spurenanalytische Methoden an.

Unter Kohlenwasserstoffen werden im Rahmen der vorliegenden Erfindung Verbindungen verstanden, die ausschließlich aus Kohlenstoff und Wasserstoff aufgebaut sind. Sofern andere Atomsorten als Kohlenstoff und Wasserstoff anwesend sind, so werden die entsprechenden Verbindungen anders bezeichnet, z. B. halogenierte Kohlenwasserstoffe.

Kohlenwasserstoffe mit 5 bis 8 Kohlenstoffatomen umfassen insbesondere gesättigte lineare aliphatische Kohlenwasserstoffe (n-Pentan, n-Hexan, n-Heptan, n-Octan), gesättigte verzweigte aliphatische Kohlenwasserstoffe, gesättigte cycloaliphatische Kohlenwasserstoffe, ungesättigte Kohlenwasserstoffe, die sich von den vorgenannten gesättigten durch rechnerische Elimination von Wasserstoff ableiten, und aromatische Kohlenwasserstoffe.

Die Gruppe der Kohlenwasserstoffe mit 5 bis 8 Kohlenstoffatomen umfasst insbesondere gesättigte oder ungesättigte cycloaliphatische Kohlenwasserstoffe und gesättigte oder ungesättigte lineare oder verzweigte aliphatische Kohlenwasserstoffe, nachfolgend gemeinsam als (cyclo)aliphatische Kohlenwasserstoffe bezeichnet, wobei die (cyclo)aliphatischen Kohlenwasserstoffe mit 5 bis 8 Kohlenstoffatomen insgesamt vorzugsweise zu höchstens 80 ppm, besonders bevorzugt höchstens 50 ppm, insbesondere höchstens 20 ppm, ganz besonders bevorzugt höchstens 10 ppm enthalten sind, jeweils bezogen auf das Gesamtgewicht des eingesetzten Monochlorbenzols einschließlich der Nebenkomponenten.

Es ist darüber hinaus bevorzugt, den Anteil von Kohlenwasserstoffen mit 6 oder 7 Kohlenstoffatomen im eingesetzten Monochlorbenzol auf insgesamt höchstens 80 ppm, bevorzugt höchstens 50 ppm, insbesondere höchstens 20 ppm, ganz besonders bevorzugt höchstens 10 ppm, insbesondere höchstens 5 ppm zu begrenzen.

Vorzugsweise weist das eingesetzte Monochlorbenzol eine Reinheit von mindestens 99,9 Gew.-%, insbesondere 99,95 Gew.-% auf, bezogen auf das Gesamtgewicht des eingesetzten Monochlorbenzols einschließlich der Nebenkomponenten. Besonders bevorzugt weist das eingesetzte Monochlorbenzol eine Reinheit von mindestens 99,99 Gew.-%, insbesondere mindestens 99,995 Gew.-%, ganz besonders bevorzugt mindestens 99,999 Gew.-% auf, bezogen auf das Gesamtgewicht des eingesetzten Monochlorbenzols einschließlich der Nebenkomponenten. Die Bestimmung des Anteils der Nebenkomponenten am eingesetzten Monochlorbenzol erfolgt im Rahmen der vorliegenden Erfindung grundsätzlich mittels gaschromatographischer Trennung und anschließender Detektion mittels Flammenionisation. Die quantitative Bestimmung der Nebenkomponenten erfolgt dabei über die Addition eines Standards.

Kohlenwasserstoffe mit 5 bis 8 Kohlenstoffatomen haben einen besonders ungünstigen Einfluss auf die Farbzahl des im erfindungsgemäßen Verfahren entstehenden 4,4'-Dichlordiphenylsulfon.

Als besonders relevant hinsichtlich des Einflusses auf die Farbzahl haben sich gesättigte cycloaliphatische Kohlenwasserstoffe der Summenformel C₆H₁₂ und C₇H₁₄, ungesättigte Kohlenwasserstoffe der Summenformel C₇H₁₂ und gesättigte aliphatische, lineare oder verzweigte Kohlenwasserstoffe der Summenformeln C₅H₁₂ und C₆H₁₄ erwiesen. Vorzugsweise beträgt der Gehalt des eingesetzten Monochlorbenzols an den im vorstehenden Satz genannten (cyclo)aliphatischen Verbindungen mit 6 oder 7 Kohlenstoffatomen insgesamt höchstens 80 ppm, besonders bevorzugt höchstens 50 ppm, insbesondere höchstens 20 ppm, ganz besonders bevorzugt höchstens 10 ppm, insbesondere höchstens 5 ppm, jeweils bezogen auf das Gesamtgewicht des eingesetzten Monochlorbenzols einschließlich der Nebenkomponenten.

Die (cyclo)aliphatischen Verbindungen mit 6 Kohlenstoffatomen, insbesondere Cyclohexan, Methylcyclopentan, Methylcyclopenten, lineares oder verzweigtes Hexan und die (cyclo)aliphatischen Verbindungen mit 7 Kohlenstoffatomen, insbesondere Methylcyclohexan, Methylcyclohexen und lineares oder verzweigtes Heptan haben dabei einen besonders ungünstigen Einfluss auf die Farbzahl des entstehenden 4,4'-Dichlordiphenylsulfon.

Darüber hinaus beträgt der Anteil an halogenierten Kohlenwasserstoffen mit 5 bis 8 Kohlenstoffatomen außer Monochlorbenzol am eingesetzten Monochlorbenzol im Rahmen der vorliegenden Erfindung vorzugsweise höchstens 100 ppm, besonders bevorzugt höchstens 50 ppm, insbesondere höchstens 20 ppm, ganz besonders bevorzugt höchstens 10 ppm, insbesondere höchstens 5 ppm, jeweils bezogen auf das Gesamtgewicht des eingesetzten Monochlorbenzols einschließlich der Nebenkomponenten. Gleichzeitig weist das eingesetzte Monochlorbenzol einen Anteil an Kohlenwasserstoffen mit 5 bis 8 Kohlenstoffatomen im oben erläuterten bevorzugten Bereich auf.

Halogenierte Kohlenwasserstoffe mit 5 bis 8 Kohlenstoffatomen außer Monochlorbenzol sind Verbindungen enthaltend Kohlenstoff, Wasserstoff und mindestens ein Halogenatom, insbesondere Chlor und/oder Brom, insbesondere Chlor. Diese Verbindungsklasse umfasst insbesondere Monobrombenzol und Dichlorbenzol.

Gegenstand der vorliegenden Erfindung ist darüber hinaus ein Verfahren zur Herstellung von 4,4'-Dichlordiphenylsulfon umfassend zumindest folgende Schritte:
(a) Aufreinigung von Monochlorbenzol unter Erhalt von Monochlorbenzol mit den erfindungsgemäßen Eigenschaften in Bezug auf die Nebenkomponenten, und anschließend
(b) Umsetzung des in Schritt (a) erhaltenen Monochlorbenzols unter Erhalt eines Gemisches umfassend 4,4'-Dichlorbenzolsulfon, und anschließend
(c) vorzugsweise Abtrennen von 4,4'-Dichlorbenzolsulfon aus dem gemäß Schritt (b) erhaltenen Gemisch.

Die einzelnen Schritte werden nachfolgend erläutert.

### Schritt (a)

Methoden zur Aufreinigung des Monochlorbenzols sind dem Fachmann an sich bekannt. Erfindungswesentlich ist, dass das in Schritt (b) eingesetzte Monochlorbenzol die oben genannten erfindungsgemäßen oder bevorzugten Eigenschaften in Bezug auf Reinheit und Nebenkomponenten aufweist.

Vorzugsweise erfolgt die Aufreinigung des Monochlorbenzols gemäß Schritt (a) destillativ. Entsprechende destillative Verfahren sind dem Fachmann ebenfalls bekannt. Es kommen insbesondere eine Batch-Destillation oder die destillative Aufreinigung mittels Trennwandkolonne in Betracht. Die destillative Aufreinigung mittels Trennwandkolonne ist besonders bevorzugt, da man mit dieser Technik in nur einem Schritt sowohl den Schwersieder- als auch den Leichtsiederteil abtrennen kann.

### Schritt (b)

Verfahren zur Herstellung von 4,4'-Dichlordiphenylsulfon ausgehend von Monochlorbenzol sind an sich bekannt und können als Verfahrensschritte (b) des vorliegenden Verfahrens implementiert werden.

Der genannte Schritt (b) betrifft die Umsetzung des in Schritt (a) erhaltenen Monochlorbenzols zu einem Gemisch enthaltend 4,4'-Dichlordiphenylsulfon (Rohprodukt). Als Nebenprodukte der Umsetzung ausgehend von Monochlorbenzol werden insbesondere 2,4'-Dichlordiphenylsulfon und/oder 3,4'-Dichlordiphenylsulfon gebildet (Fehlisomere des 4,4'-Dichlordiphenylsulfon). Darüber hinaus werden im Allgemeinen 2-Chlorbenzolsulfonsäure, 3-Chlorbenzolsulfonsäure und/oder 4-Chlorbenzolsulfonsäure gebildet. Die oben genannten Kohlenwasserstoffe mit 5 bis 8 Kohlenstoffatomen als Nebenkomponenten von Monochlorbenzol führen darüber hinaus zu einem komplexen Spektrum farbgebender Verbindungen im genannten Rohprodukt.

Üblicherweise erfolgt die Umsetzung von Monochlorbenzol mit einem Sulfonierungsmittel unter Bildung von 4-Chlorbenzolsulfonsäure. Hierbei entstehen allerdings unvermeidlich Fehlisomere von 4-Monochlorbenzolsäure als an sich unerwünschte Nebenprodukte. Anschließend werden die 4-Chlorbenzolsulfonsäure und deren Isomere 2-Chlorbenzolsulfonsäure und/oder 3-Chlorbenzolsulfonsäure mit Monochlorbenzol zu 4,4'-Dichlordiphenylsulfon umgesetzt, wobei die oben genannten Fehlisomere von 4,4'-Dichlordiphenylsulfon gebildet werden. Die Bildung von Monochlorbenzolsulfonsäure kann auch als Zwischenprodukt erfolgen, das nicht isoliert wird.

In einer bevorzugten ersten Ausführungsform erfolgt die Herstellung von 4,4'-Dichlordiphenylsulfon durch Reaktion von 4-Chlorbenzolsäure mit Monochlorbenzol. Die Reaktion wird vorzugsweise in einer Gegenstromkolonne durchgeführt, wobei das Reaktionswasser durch den im Sumpf der Kolonne gasförmig zugegebenen Aromaten kontinuierlich über Kopf ausgestrippt wird. Für die Synthese von 4,4'-Dichlordiphenylsulfon kann am Kopf der Kolonne 4-Chlorbenzolsäure oder auch Schwefelsäure zugegeben werden. Letztere reagiert in der Kolonne mit Monochlorbenzol zuerst zu Monochlorbenzolsulfonsäure, welche anschließend ebenfalls mit Monochlorbenzol zu Dichlordiphenylsulfon reagiert. Das entsprechende Verfahren ist beispielsweise in der US 2,593,001 beschrieben, deren Inhalt hiermit in vollem Umfang einbezogen sei.

In einer zweiten bevorzugten Ausführungsform erfolgt die Herstellung von Dichlordiphenylsulfon unter Verwendung von SO₃, Dimethylsulfat und Monochlorbenzol. Vorzugsweise lässt man zuerst bei moderaten Bedingungen SO₃ und Dimethylsulfat im molaren Verhältnis von 2 zu 1 reagieren. Ein Teil des SO₃ reagiert dabei mit Dimethylsulfat zum entsprechenden Pyrosulfat. Das restliche SO₃ verbleibt gelöst in der entstandenen Flüssigkeit. Diese Mischung wird anschließend bei Temperaturen unter 100°C zu 2 mol Monochlorbenzol pro 2 mol SO₃ und 1 mol Dimethylsulfat zugegeben. Aus dem gelösten SO₃, dem Dimethylpyrosulfat und dem Monochlorbenzol entstehen 1 mol Dichlordiphenylsulfon und 2 mol Monomethylsulfat. Die Reaktionsmischung wird anschließend in Wasser geleitet. Es fällt Dichlordiphenylsulfon aus. Dieses wird abfiltriert und getrocknet. Das entsprechende Verfahren wird beispielsweise in der US 2,971,985 beschrieben, deren Inhalt hiermit in vollem Umfang einbezogen sei.

Dem Fachmann ist bekannt, dass er die vorstehend erläuterten Umsetzungen mit anderen Aufarbeitungsverfahren als den vorstehend aufgeführten kombinieren kann.

### Schritt (c)

Im Rahmen von Schritt (c) erfolgt vorzugsweise die Abtrennung von 4,4'-Dichlordiphenylsulfon aus dem gemäß Schritt (b) erhaltenen Gemisch, d. h. die Aufarbeitung des Rohproduktes, welches das gewünschte Reaktionsprodukt sowie Nebenprodukte enthält.

Geeignete Verfahren zur Aufarbeitung des Rohproduktes sind dem Fachmann an sich bekannt.

In einer Ausführungsform erfolgt die Auftrennung des Reaktionsgemischs durch Zugabe von Wasser und der Separierung der beiden entstehenden flüssigen Phasen. Die wässrige Phase enthält nicht umgesetzte Monochlorbenzolsulfonsäure. Das Wasser wird abgedampft und die Monochlorbenzolsulfonsäure als Einsatzstoff zurückgewonnen. Aus der organischen Phase, die überwiegend aus Monochlorbenzol und Dichlordiphenylsulfon besteht, kann Dichlordiphenylsulfon isoliert werden. Ein entsprechendes Verfahren wird beispielsweise in der US 4,937,387 beschrieben, deren Inhalt hiermit in vollem Umfang einbezogen sei.

Die Abtrennung von 4,4'-Dichlordiphenylsulfon aus dem Rohprodukt kann beispielsweise chromatographisch erfolgen. Vorzugsweise erfolgt die Abtrennung durch Umkristallisation wie beispielsweise in der EP 279 387 beschrieben, deren Inhalt hiermit in vollem Umfang einbezogen wird.

### Beispiele

### Beispiel 1

Das in Beispiel 1 eingesetzte Monochlorbenzol enthielt gemäß gaschromatographischer Trennung und anschließender Detektion mittels Flammenionisation folgende Nebenkomponenten: 30 ppm Methylcyclohexan und 10 ppm Methylcyclohexen.

126,1 g (1 mol) Dimethylsulfat wurden unter Ausschluss von Luftfeuchtigkeit auf 70-75°C erhitzt und es wurden bei dieser Temperatur 80,1 g (1 mol) flüssiges Schwefeltrioxid zugegeben. Man ließ 30 min bei dieser Temperatur nachrühren und kühlte anschließend auf 20°C ab. Es wurden weitere 80,1 g (1 mol) flüssiges Schwefeltrioxid so zugegeben, dass die Temperatur 30°C nicht überschreitet. Die Reaktionsmischung wurde innerhalb 20 min zu 225,1 g (2 mol) auf 50°C vorgeheiztem Monochlorbenzol gegeben. Anschließend wurde noch 1 h bei 50°C gerührt. Nach Abkühlen erhielt man eine gelblich-orangene Lösung.

### Beispiel 2

Das in Beispiel 2 eingesetzte Monochlorbenzol enthielt gemäß gaschromatographischer Trennung und anschließender Detektion mittels Flammenionisation folgende Nebenkomponenten: Kohlenwasserstoffe mit von 5 bis 8 Kohlenstoffatomen insgesamt < 1 ppm.

126,1 g (1 mol) Dimethylsulfat wurden unter Ausschluss von Luftfeuchtigkeit auf 70-75°C erhitzt und es wurden bei dieser Temperatur 80,1 g (1 mol) flüssiges Schwefeltrioxid zugegeben. Man ließ 30 min bei dieser Temperatur nachrühren und kühlte anschließend auf 20°C ab. Es wurden weitere 80,1 g (1 mol) flüssiges Schwefeltrioxid so zugegeben, dass die Temperatur 30°C nicht überschreitet. Die Reaktionsmischung wurden innerhalb 20 min zu 225,1 g (2 mol) auf 50°C vorgeheiztem Monochlorbenzol gegeben. Anschließend wurde noch 1 h bei 50°C gerührt. Nach Abkühlen erhielt man eine hellgelbe Lösung.

### Beispiel 3

126,1 g (1 mol) Dimethylsulfat wurden unter Ausschluss von Luftfeuchtigkeit auf 70-75°C erhitzt und es wurden bei dieser Temperatur 80,1 g (1 mol) flüssiges Schwefeltrioxid zugegeben. Man ließ 30 min bei dieser Temperatur nachrühren und kühlte anschließend auf 20°C ab. Es wurden weitere 80,1 g (1 mol) flüssiges Schwefeltrioxid so zugegeben, dass die Temperatur 30°C nicht überschreitet. Die Reaktionsmischung wurde innerhalb 20 min zu einer Mischung von 1,68 g (0,02 mol) Cyclohexan und 225,1 g (2 mol) auf 50°C vorgeheiztem Monochlorbenzol (identisch zu Beispiel 1 gegeben. Anschließend wurde noch 1 h bei 50°C gerührt. Nach Abkühlen erhielt man eine dunkel-orangene Lösung.

Durch das erfindungsgemäße Verfahren wird ein Rohprodukt von 4,4'-Dichlordiphenylsulfon erhalten, das eine deutlich geringere Farbzahl und somit eine deutlich höhere Reinheit aufweist. Das erfindungsgemäß erhaltene Rohprodukt von 4,4'-Dichlordiphenylsulfon kann bekannten Verfahren zur Aufarbeitung und Gewinnung von reinem 4,4'-Dichlordiphenylsulfon unterzogen werden. Die Aufarbeitung kann mit geringerer Komplexität erfolgen, insbesondere in dem die bekannten Verfahren schneller und/oder in einer geringeren Wiederholungszahl durchgeführt werden.

### Beispiel 4

Das in Beispiel 4 eingesetzte Monochlorbenzol enthielt gemäß gaschromatographischer Trennung und anschließender Detektion mittels Flammenionisation folgende Nebenkomponenten: 20 ppm Hexan, 20 ppm Cyclohexan, 10 ppm Methylcyclohexan, 10 ppm Brombenzol und 10 ppm Dichlorbenzol.

Zu 1914 g (17 mol) Monochlorbenzol wurden unter Ausschluss von Luftfeuchtigkeit bei maximal 45°C 680 g (8,5 mol) frisch destilliertes Schwefeltrioxid hinzugegeben. Die resultierende dunkel-graubraune Lösung (Chlorbenzolsulfonsäure in Monochlorbenzol) kann zur Synthese von Dichlordiphenylsulfon, beispielsweise nach US 2,593,001, eingesetzt werden.

### Beispiel 5

Das in Beispiel 5 eingesetzte Monochlorbenzol wurde erhalten, indem das in Beispiel 1 eingesetzte Monochlorbenzol zusätzlich anschließend in einer Batch-Destillationskolonne über P₂O₅ destilliert und somit aufgereinigt wurde. Das in Beispiel 5 eingesetzte Monochlorbenzol enthielt gemäß gaschromatographischer Trennung und anschließender Detektion mittels Flammenionisation folgende Nebenkomponenten: Kohlenwasserstoffe mit von 5 bis 8 Kohlenstoffatomen < 1 ppm.

Zu 1914 g (17 mol) Monochlorbenzol wurden unter Ausschluss von Luftfeuchtigkeit bei maximal 45°C 680 g (8,5 mol) frisch destilliertes Schwefeltrioxid hinzugegeben. Die schwach braune Lösung (Chlorbenzolsulfonsäure in Monochlorbenzol) kann zur Synthese von Dichlordiphenylsulfon, beispielsweise nach US 2,593,001, eingesetzt werden.

### Beispiel 6

Zu einer Mischung von 1914 g (17 mol) Monochlorbenzol (identisch zu Beispiel 5) und 8,4 g (0,1 mol) Cyclohexan wurden unter Ausschluss von Luftfeuchtigkeit bei maximal 45°C 680 g (8,5 mol) frisch destilliertes Schwefeltrioxid hinzugegeben. Die resultierende Lösung (Chlorbenzolsulfonsäure in Monochlorbenzol) war schwarz-braun.

## Patentansprüche

1. Verfahren zur Herstellung von 4,4'-Dichlordiphenylsulfon ausgehend von Monochlorbenzol, wobei der Gehalt des eingesetzten Monochlorbenzols an Kohlenwasserstoffen mit von 5 bis 8 Kohlenstoffatomen höchstens 100 ppm, bezogen auf das Gesamtgewicht des eingesetzten Monochlorbenzols einschließlich der Nebenkomponenten, beträgt.

2. Verfahren nach Anspruch 1, wobei der Gehalt des eingesetzten Monochlorbenzols an halogenierten Kohlenwasserstoffen mit 5 bis 8 Kohlenstoffatomen außer Monochlorbenzol höchstens 50 ppm und der Gehalt des eingesetzten Monochlorbenzols an Kohlenwasserstoffen mit von 5 bis 8 Kohlenstoffatomen höchstens 50 ppm, bezogen auf das Gesamtgewicht des eingesetzten Monochlorbenzols einschließlich der Nebenkomponenten, beträgt.

3. Verfahren nach Anspruch 1 oder 2, wobei der Gehalt des eingesetzten Monochlorbenzols an Kohlenwasserstoffen mit von 5 bis 8 Kohlenstoffatomen höchstens 20 ppm, bezogen auf das Gesamtgewicht des eingesetzten Monochlorbenzols einschließlich der Nebenkomponenten, beträgt.

4. Verfahren nach einem oder mehreren der Ansprüche 1 bis 3, wobei der Gehalt des eingesetzten Monochlorbenzols an Kohlenwasserstoffen mit von 5 bis 8 Kohlenstoffatomen höchstens 10 ppm, bezogen auf das Gesamtgewicht des eingesetzten Monochlorbenzols einschließlich der Nebenkomponenten, beträgt.

5. Verfahren nach einem oder mehreren der Ansprüche 1 bis 4, wobei das eingesetzte Monochlorbenzol eine Reinheit von mindestens 99,9 Gew.-%, bezogen auf das Gesamtgewicht des eingesetzten Monochlorbenzols einschließlich der Nebenkomponenten, aufweist.

6. Verfahren nach einem oder mehreren der Ansprüche 1 bis 5, wobei das eingesetzte Monochlorbenzol eine Reinheit von mindestens 99,99 Gew.-% aufweist.

7. Verfahren nach einem oder mehreren der Ansprüche 1 bis 6, wobei der Gehalt des eingesetzten Monochlorbenzols an (cyclo)aliphatischen Kohlenwasserstoffen mit 6 oder 7 Kohlenstoffatomen insgesamt höchstens 50 ppm, bezogen auf das Gesamtgewicht des eingesetzten Monochlorbenzols einschließlich der Nebenkomponenten, beträgt.

8. Verfahren nach einem oder mehreren der Ansprüche 1 bis 7, wobei der Gehalt des eingesetzten Monochlorbenzols an (cyclo)aliphatischen Kohlenwasserstoffen mit 6 oder 7 Kohlenstoffatomen insgesamt höchstens 10 ppm, bezogen auf das Gesamtgewicht des eingesetzten Monochlorbenzols einschließlich der Nebenkomponenten, beträgt.

9. Verfahren zur Herstellung von 4,4'-Dichlordiphenylsulfon umfassend
(a) Aufreinigung von Monochlorbenzol unter Erhalt von Monochlorbenzol wie in einem oder mehreren der Ansprüche 1 bis 8 definiert, und anschließend
(b) Umsetzung des in Schritt (a) erhaltenen Monochlorbenzols zu einem Gemisch enthaltend 4,4'-Dichlordiphenylsulfon.

10. Verfahren nach Anspruch 9, wobei in Anschluss an Schritt (b) gemäß Schritt (c) die Abtrennung von 4,4'-Dichlordiphenylsulfon aus dem gemäß Schritt (b) erhaltenen Gemisch erfolgt.

11. Verfahren nach Anspruch 9 oder 10, wobei die Aufreinigung des Monochlorbenzols gemäß Schritt (a) destillativ erfolgt.

12. Verfahren nach einem oder mehreren der Ansprüche 9 bis 11, wobei die Aufreinigung des Monochlorbenzols gemäß Schritt (a) destillativ in einer Trennwandkolonne erfolgt.

13. Verwendung von Monochlorbenzol zur Herstellung von 4,4'-Dichlorphenylsulfon, wobei der Gehalt des Monochlorbenzols an Kohlenwasserstoffen mit 5 bis 8 Kohlenstoffatomen höchstens 100 ppm beträgt, jeweils bezogen auf das Gesamtgewicht des eingesetzten Monochlorbenzols einschließlich der Nebenkomponenten.
